(19) Europäisches Patentamt
European Patent Office
Office européen des brevets

(11) Publication number: 0 073 569 A1

(12) EUROPEAN PATENT APPLICATION

(21) Application number: 82303944.1

(22) Date of filing: 26.07.82

(51) Int. Cl.³: C 07 C 29/40, C 07 C 33/28, C 07 C 33/24, C 07 C 35/17, C 07 D 307/44, C 07 D 333/16, C 07 B 17/00

(30) Priority: 27.07.81 JP 118253/81

(43) Date of publication of application: 09.03.83 Bulletin 83/10

(84) Designated Contracting States: CH DE FR GB IT LI

(71) Applicant: SUMITOMO CHEMICAL COMPANY, LIMITED, 15 Kitahama 5-chome Higashi-ku, Osaka-shi Osaka-fu (JP)

(72) Inventor: Kono, Nobuharu, 9-7-24, Hommachi, Toyonaka Osaka (JP)
Inventor: Takisawa, Yukihisa, 1-2-40, Hirata, Ibaraki Osaka (JP)
Inventor: Saito, Kenji, No. 321,2-10-3 Sonehigashi-machi, Toyonaka Osaka (JP)
Inventor: Yamachika, Hiroshi, 2-1, Kuwata-cho, Ibaraki Osaka (JP)
Inventor: Takahashi, Kunitoshi, 9-8-68 Hommachi, Toyonaka Osaka (JP)

(74) Representative: Allard, Susan Joyce et al, BOULT, WADE & TENNANT 27 Furnival street, London EC4A 1PQ (GB)

(54) **Process for the continuous production of alcohols.**

(57) A process for the continuous production of an alcohol from an organic halide, metallic magnesium and an organic carbonyl compound according to the Grignard reaction, which process comprises continuously supplying a portion of an intended amount of the organic halide and an intended amount of the metallic magnesium with an inert solvent to a first reaction zone whereby the reaction between the organic halide and the metallic magnesium proceeds to give the Grignard reagent, and continuously passing the reaction mixture comprising the Grignard reagent and the unreacted metallic magnesium from the first reaction zone to a second reaction zone to which the remaining portion of the organic halide and an intended amount of the organic carbonyl compound, optionally with an inert solvent, are continuously supplied whereby the Grignard reaction proceeds to give the Grignard reaction product, followed by subjecting the reaction mixture comprising the Grignard reaction product from the second reaction zone to hydrolysis to give the alcohol.

EP 0 073 569 A1

PROCESS FOR THE CONTINUOUS PRODUCTION OF ALCOHOLS

The present invention relates to a process for the continuous production of alcohols. More particularly, it relates to a process for the continuous production of alcohols from organic halides, metallic magnesium and organic carbonyl compounds.

It is known to produce alcohols by subjecting organic carbonyl compounds, such as aldehydes or ketones, to the Grignard reaction to give Grignard reaction products, which are then hydrolyzed. When the Grignard reaction is carried out in a batch process, temperature control is extremely difficult, and especially at an early stage, almost impossible since the reaction is exothermic. Furthermore, the Grignard reaction is usually carried out in a solvent such as diethyl ether or tetrahydrofuran, which is inflammable. These factors make it difficult to carry out the Grignard reaction in a batch process on a large scale.

Usually, the production of a Grignard reaction product according to the Grignard reaction is effected in two steps; i.e. the entire amount of an organic halide and of metallic magnesium are first reacted to make a Grignard reagent and then the Grignard reagent is reacted with the entire amount of an organic carbonyl compound. When a highly active compound having an unsaturated bond at the β-position is used as the organic halide,

a considerable amount of by-products are produced, which may result from the following Wurtz reaction:

$$R\text{-}X + R\text{-}MgZ \longrightarrow R\text{-}R + MgX_2$$

, and the yield of the desired Grignard reaction product is lowered.

In order to overcome the above drawbacks of the Grignard reaction, we have found that when a portion of an intended amount of an organic halide and an intended amount of metallic magnesium are first subjected to reaction and then the remaining portion of the organic halide and an intended amount of an organic carbonyl compound are subjected to reaction in the presence of the reaction mixture comprising the Grignard reagent and the unreacted metallic magnesium obtained in the foregoing step, the Grignard reaction proceeds smoothly to give the Grignard reaction product in an excellent yield with less formation of by-products.

According to the present invention, there is provided a process for the continuous production of an alcohol from an organic halide, metallic magnesium and an organic carbonyl compound according to the Grignard reaction, which process comprises continuously supplying a portion of an intended amount of the organic halide and an intended amount of the metallic magnesium with an inert solvent to a first reaction zone whereby the reaction between the organic halide and the metallic

magnesium proceeds to give the Grignard reagent, and continuously passing the reaction mixture comprising the Grignard reagent and the unreacted metallic magnesium from the first reaction zone to a second reaction zone to which the remaining portion of the organic halide and an intended amount of the organic carbonyl compound, optionally with an inert solvent, are continuously supplied whereby the Grignard reaction proceeds to give the Grignard reaction product, followed by subjecting the reaction mixture comprising the Grignard reaction product from the second reaction zone to hydrolysis to give the alcohol.

As will be understood from the above, it is essential in the process of the present invention that a portion of an intended amount of an organic halide and an intended amount of metallic magnesium are continuously supplied to the first reaction zone, and the reaction mixture obtained in the first reaction zone as well as the remaining portion of the organic halide and an intended amount of the organic carbonyl compound are supplied to the second reaction zone. By this process, the Grignard reagent is formed in the first reaction zone, and simultaneously the unreacted metallic magnesium is activated therein. As the result, the reaction mixture transferred from the first reaction zone to the second reaction zone comprises the Grignard reagent and the activated metallic magnesium, and the Grignard reaction between the Grignard

reagent and the organic carbonyl compound supplied to the second reaction zone proceeds smoothly with supplemental and continuous production of the Grignard reagent _in situ_ from the activated metallic magnesium and the organic halide supplied thereto. Since the Grignard reaction is usually much influenced by the purity and surface state of metallic magnesium, the supply of the metallic magnesium activated in the first reaction zone to the second reaction zone can contribute greatly to the smooth and stabilized procedure of the Grignard reaction in the second reaction zone.

In the second reaction zone, the following Grignard reaction proceeds with the organic halide and the organic carbonyl compound in the presence of the reaction mixture comprising the Grignard reagent and the activated metallic magnesium from the first reaction zone:

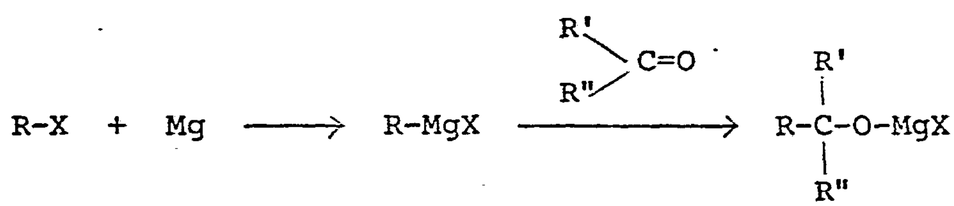

The term "intended amount" as used herein means the amount of any starting material as usually and conventionally employed for the production of the desired product. It can be calculated theoretically but is not necessarily required to be exactly equal to the calculated theoretical amount. In other words, any of the starting materials may be employed in more or less a larger amount than the theoretical amount insofar as any unfavorable influence on the procedure of the reaction and/or the desired product is not caused thereby. The term "portion" used in connection with the amount of the organic halide is intended to mean any amount sufficient to activate the metallic magnesium supplied to the first reaction zone, and such amount is preferred to be as small as possible.

Although the process of the invention is generally applicable to the production of alcohols from organic halides, metallic magnesium and organic carbonyl compounds according to the Grignard reaction, a remarkable advantage is observed on its application to the case wherein the organic halides have an unsaturated bond at the β-position, because the yields of the desired alcohols are much improved in comparison with those attained by conventional procedures.

Thus, the organic halides to which this invention is advantageously applied may be represented by the formula:

$$R_1\text{-}X \qquad (I)$$

wherein $R_1$ is an arylmethyl group or an alkenyl or alkynyl group having an unsaturated bond at the β-position and X is a halogen atom.

The organic carbonyl compounds may be represented by the formula:

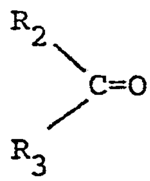

(II)

wherein $R_2$ is a hydrogen atom, an alkyl group, an alkenyl group, a phenyl group, a naphthyl group, a benzyl group, a furyl group or a thienyl group and $R_3$ is an alkyl group, an alkenyl group, a phenyl group, a naphthyl group, a benzyl group, a furyl group or a thienyl group.

When the organic halides (I) and the organic carbonyl compounds (II) are used, the Grignard reagents are representable by the formula:

$R_1$-MgX (III)

wherein $R_1$ and X are each as defined above, the Grignard reaction products are representable by the formula:

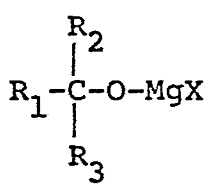

(IV)

wherein $R_1$, $R_2$, $R_3$ and X are each as defined above, and the desired alcohols are representable by the formula:

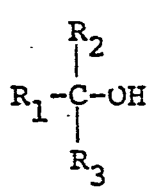

(V)

wherein $R_1$, $R_2$ and $R_3$ are each as defined above.

In the above definitions, the arylmethyl group represented by $R_1$ usually has not more than 15 carbon atoms and examples thereof are benzyl, p-methylbenzyl, naphthylbenzyl and naphthylmethyl. The alkenyl

or alkynyl group having an unsaturated bond at the β-position represented by $R_1$ is usually one having not more than 8 carbon atoms. Specific examples are allyl, α-methylallyl, crotyl, α-methylcrotyl, propargyl and α-methylpropargyl . The alkyl group and the alkenyl group represented by $R_2$ or $R_3$ may be those having not more than 12 carbon atoms, and examples thereof include methyl, ethyl, propyl, butyl, pentyl, octyl, allyl and crotyl. The halogen atom may be, for example, chlorine, bromine or iodine.

Specific examples of the organic halides (I) are allyl chloride, allyl bromide, α-methylallyl chloride, α-methylallyl bromide, crotyl chloride, crotyl bromide, α-methylcrotyl chloride, propargyl chloride, α-methylpropargyl chloride, benzyl chloride, benzyl bromide, benzyl iodide, p-methylbenzyl chloride and naphthylmethyl chloride.

Specific examples of the organic carbonyl compounds (II) are aldehydes (e.g. acetaldehyde, propionaldehyde, cyclohexylaldehyde, isobutylaldehyde, crotonaldehyde, 2-cyclopentenylaldehyde, benzaldehyde, furfural, 5-methylfurfural, 2-thiophenecarboxaldehyde or phenylacetaldehyde and ketones (e.g. acetone, methyl ethyl ketone, methyl isobutyl ketone, cyclohexanone, acetophenone or benzophenone.

The metallic magnesium is preferred to be used in the form of small particles for its smooth transfer from the first reaction zone to the second reaction zone. However,

particles which are too small may be easily oxidized and adversely affect the reaction.

The inert solvent preferably employed is a cyclic or non-cyclic ether (e.g. tetrahydrofuran, tetrahydropyrane, diethyl ether, dibutyl ether, anisole, tetrahydrofurfuryl ethyl ether, ethylene glycol dimethyl ether, diethylene glycol diethyl ether or glycerol trimethyl ether . In combination with these ethers, other solvents inert to the Grignard reaction may be used such as benzene, toluene, xylene, n-hexane, cyclohexane and kerosene. In general, the use of a solvent(s) which can dissolve the Grignard reagent (III) and the Grignard reaction product (IV) therein is favorable.

A promoter for the Grignard reaction may be additionally introduced into the first reaction zone and/or the second reaction zone. Specific examples of a promoter are iodine, bromine, ethyl bromide, methyl idodide, ethyl iodide, zinc bromide, mercury iodide and cupric chloride.

The intended amounts of the starting materials may be determined in the following manner: the amount of one material to be entirely consumed is determined first, and the other materials are used in slightly over their theoretical amounts corresponding to the said determined amount. The material to be entirely consumed may be appropriately chosen by taking into consideration the influence onto the operation(s) in the subsequent step(s), and the reaction vessels, etc. For instance, the choice of

the metallic magnesium as the material to be entirely consumed can avoid removal or recovery of the unreacted metallic magnesium. In general, the molar proportion of the organic halide (I), metallic magnesium and the organic carbonyl compound (II) may be 0.7 - 2 : 0.7 - 2 : 1, preferably 0.9 - 1.5 : 0.9 - 1.5 : 1.

The amount of the organic halide (I) to be supplied to the first reaction zone is to be sufficient to activate the metallic magnesium. In order to minimize the loss of the organic halide (I) due to the Wurtz reaction, however, it is preferred to be as small as possible. Thus, it is preferably 1 to 20 % by weight, more preferably 2 to 10 % by weight, of the entire amount of the organic halide (I) to be used in the whole reaction zones.

The amount of the inert solvent to be supplied to the first reaction zone may be such that the reaction mixture including the unreacted metallic magnesium can be effectively stirred in the first reaction zone and also the unreacted metallic magnesium can be smoothly transferred from the first reaction zone to the second reaction zone.

Practically, the process of the invention may be carried out by the use of at least two reactors, of which the first one corresponds to the first reaction zone and the other(s) correspond (s) to the second reaction zone. Each reactor is preferably of the tank type. Into the first reactor, the entire amount of metallic magnesium, a partial

amount of the organic halide (I) and an appropriate amount of inert solvent are continuously charged separately or in combination, whereby the reaction proceeds between the metallic magnesium and the organic halide (I) to give the Grignard reagent (III) in an amount corresponding to the charged amount of the organic halide (I). The resulting mixture comprising the unreacted metallic magnesium in addition to the Grignard reagent (III) flows out continuously into the second reactor, to which the rest of the organic halide (I), an entire amount of the organic carbonyl compound (II) and optionally an appropriate amount of the inert solvent are continuously charged separately, or in combination. The inert solvent to be optionally supplied to the second reactor is preferably the same as that used in the first reactor in view of ease of post-treatment. In the second reactor, the organic carbonyl compound (II) is reacted with the Grignard reagent (III) formed in the first reactor and also freshly formed in the second reactor to yield the Grignard reaction product (IV) at a rate corresponding to the feed rate of the starting materials. Alternatively, the materials to be charged into the second reactor may be divided into several portions and charged into several succeeding reactors.

Throughout the above operations, the feed rate of each starting material in each reactor should be controlled appropriately so that no starting material will be

charged in large excess. The residence time varies with the reaction conditions such as the type of reactant and the reaction temperature and is generally from 0.2 to 4 hours, preferably from 0.5 to 2 hours in the first reaction zone and generally from 0.5 to 8 hours, preferably from 1 to 5 hours in the second reaction zone.

The reaction temperature in the first and second reaction zones is usually from -20°C to the boiling point of the inert solvent to be used, preferably from 0 to 50°C. Too low a reaction temperature increases the viscosity of the reaction mixture and requires more energy to remove the generated heat. The reaction pressure is not critical. Under reduced pressure, heat can be removed by reflux, and thereby the reaction temperature can be controlled.

The Grignard reaction product (IV) obtained as above is then hydrolyzed in a continuous or batch process to yield the desired alcohol (V). The hydrolysis may be carried out by a conventional method, for example, treatment with an aqueous solution of ammonium chloride, hydrochloric acid, sulfuric acid or acetic acid, usually at a temperature of -10°C to the reflux temperature, preferably of 0 to 60°C. The thus produced alcohol (V) may be purified, for example, by distillation.

The present invention will be hereinafter explained in more detail by the following Examples.

Example 1

Into a 200 ml volume first reactor equipped with a cooling coil and baffles, 15 to 40 mesh flaky metallic magnesium (feed rate: 20 g/hr), allyl chloride (feed rate: 41 g/hr), tetrahydrofuran (feed rate: 41 g/hr) and toluene (feed rate: 123 g/hr) were continuously introduced, and the reaction was carried out at 35 to 40°C under stirring with a residence time of about 1 hour. The reaction mixture including unreacted metallic magnesium continuously flowed out from an overflow outlet was led into a 1000 ml volume second reactor equipped with a cooling coil and baffles. Into the second reactor, allyl chloride (feed rate: 64 g/hr), 5-methylfurfural (feed rate: 82 g/hr) and toluene (feed rate: 164 g/hr) were introduced continuously, and the reaction was carried out at 35 to 40°C under stirring with a residence time of about 2 hours. The reaction mixture continuously flowed out from an overflow outlet was subjected to hydrolysis with a 6.2 % aqueous solution of sulfuric acid at 30 to 35°C to obtain 5-methyl-α-(2'-propenyl)furfuryl alcohol in a 98 % yield.

Example 2

Into the first reactor as used in Example 1, metallic magnesium (feed rate: 19 g/hr), propargyl bromide (feed rate: 9.2 g/hr) and diethyl ether (feed rate: 250 g/hr) were continuously introduced, and the reaction was carried out at 30 to 35°C under stirring with a residence time of about 30 minutes. The reaction mixture including unreacted metallic magnesium flowed out from the overflow

outlet was led into the second reactor as used in Example 1. Into the second reactor, propargyl bromide (feed rate: 82.4 g/hr) and α-thiophenecarboxaldehyde (feed rate: 78.4 g/hr) were continuously introduced, and the reaction was carried out at 30 to 35°C under stirring with a residence time of 2 hours. The reaction mixture flowed out from the overflow outlet was subjected to hydrolysis with a 20 % aqueous solution of ammonium chloride at 30 to 35°C to obtain α-propargylthienylmethanol in a 90 % yield.

Example 3

Into the first reactor as used in Example 1, metallic magnesium (feed rate: 19 g/hr), benzyl bromide (feed rate: 7.2 g/hr), tetrahydrofuran (feed rate: 80 g/hr) and xylene (feed rate: 80 g/hr) were continuously introduced, and the reaction was carried out at 40 to 45°C under stirring with a residence time of about 1 hour. The reaction mixture including unreacted metallic magnesium flowed out from the overflow outlet was led into the second reactor as used in Example 1. Into the second reactor, benzyl bromide (feed rate: 136.4 g/hr) and acetophenone (feed rate: 84 g/hr) were continuously introduced, and the reaction was carried out at 40 to 45°C under stirring with a residence time of about 2.5 hours. The reaction mixture flowed out from the overflow outlet was subjected to hydrolysis with a 6.2 % aqueous solution of sulfuric acid at 40 to 45°C to obtain α-methyl-α-phenyl-β-phenethyl alcohol in a 80 % yield.

Example 4

Into the first reactor as used in Example 1, metallic magnesium (feed rate: 19 g/hr), allyl chloride (feed rate: 6.4 g/hr) and tetrahydrofuran (feed rate: 70 g/hr) were continuously introduced, and the reaction was carried out at 30 to 35°C under stirring with a residence time of about 30 minutes. The reaction mixture including unreacted metallic magnesium flowed out from the overflow outlet was led into the second reactor as used in Example 1. Into the second reactor, allyl chloride (feed rate: 57.9 g/hr), cyclohexanone (feed rate: 45 g/hr) and tetrahydrofuran (feed rate: 100 g/hr) were continuously introduced, and the reaction was carried out at 30 to 35°C under stirring with a residence time of 3 hours. The reaction mixture flowed out from the overflow outlet was subjected to hydrolysis with a 20 % aqueous solution of ammonium chloride at 30 to 35°C to obtain 1-allylcyclohexanol in a 93 % yield.

Example 5

Into the first reactor as used in Example 1, metallic magnesium (feed rate: 19 g/hr), propargyl bromide (feed rate: 5.0 g/hr) and tetrahydrofuran (feed rate: 170 g/hr) were continuously introduced, and the reaction was carried out at 35 to 40°C under stirring with a residence time of about 1 hour. The reaction mixture including unreacted metallic magnesium flowed out from the overflow outlet was led into the second reactor as used in Example 1. Into the second reactor, propargyl bromide (feed rate: 95.8

g/hr) and benzophenone (feed rate: 127.5 g/hr) were continuously introduced, and the reaction was carried out at 35 to 40°C under stirring with a residence time of about 2 hours. The reaction mixture flowed out from the overflow outlet was subjected to hydrolysis with a 6.2 % aqueous solution of ammonium chloride at 35 to 40°C to obtain α,α-diphenyl-1-butynyl alcohol in a 88 % yield.

Example 6

Into the first reactor as used in Example 1, metallic magnesium (feed rate: 19 g/hr), benzyl bromide (feed rate: 15.5 g/hr) and diethyl ether (feed rate: 50 g/hr) were continuously introduced, and the reaction was carried out at 40 to 45°C under stirring with a residence time of about 1 hour. The reaction mixture including unreacted metallic magnesium flowed out from the overflow outlet was led into the second reactor as used in Example 1. Into the second reactor, benzyl bromide (feed rate: 139.9 g/hr) and acetone (feed rate: 40.6 g/hr) were continuously introduced, and the reaction was carried out at 40 to 45°C under stirring with a residence time of about 3 hours. The reaction mixture flowed out from the overflow outlet was subjected to hydrolysis with a 6.2 % aqueous solution of sulfuric acid at 40 to 45°C to obtain α,α-dimethylphenethyl alcohol in a 85 % yield.

Comparative Example 1

Into the first reactor as used in Example 1, 15 to 40 mesh flaky metallic magnesium (feed rate: 10 g/hr),

allyl chloride (feed rate: 34 g/hr) and tetrahydrofuran (feed rate: 136 g/hr) were continuously introduced, and the reaction was carried out at 35 to 40°C under stirring with a residence time of about 1 hour. The reaction mixture including unreacted metallic magnesium continuously flowed out from the overflow outlet was led into the second reactor (1000 ml) as used in Example 1. Into the second reactor, 5-methylfurfural (feed rate: 41 g/hr) and toluene (feed rate: 272 g/hr) were continously introduced, and the reaction was carried out at 35 to 40°C under stirring with a residence time of about 2 hours. The reaction mixture flowed out from the overflow outlet was subjected to hydrolysis with a 6.2 % aqueous solution of sulfuric acid at 35 to 40°C to obtain 5-methyl-α-(2'-propenyl)furfuryl alcohol in a 65.1 % yield.

CLAIMS:

1. A process for the continuous production of an alcohol from an organic halide, metallic magnesium and an organic carbonyl compound according to the Grignard reaction, which process comprises continuously supplying a portion of an intended amount of the organic halide and an intended amount of the metallic magnesium with an inert solvent to a first reaction zone whereby the reaction between the organic halide and the metallic magnesium proceeds to give the Grignard reagent, and continuously passing the reaction mixture comprising the Grignard reagent and the unreacted metallic magnesium from the first reaction zone to a second reaction zone to which the remaining portion of the organic halide and an intended amount of the organic carbonyl compound, optionally with an inert solvent, are continuously supplied whereby the Grignard reaction proceeds to give the Grignard reaction product, followed by subjecting the reaction mixture comprising the Grignard reaction product from the second reaction zone to hydrolysis to give the alcohol.

2. A process as claimed in Claim 1, wherein the organic halide is represented by the formula:

$$R_1-X$$

wherein $R_1$ is an arylmethyl group or an alkenyl or alkynyl

group having an unsaturated bond at the β-position and X is a halogen atom, the organic carbonyl compound is represented by the formula:

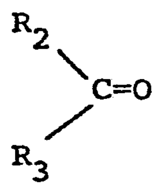

wherein $R_2$ is a hydrogen atom, an alkyl group, an alkenyl group, a phenyl group, a naphthyl group, a benzyl group, a furyl group or a thienyl group and $R_3$ is an alkyl group, an alkenyl group, a phenyl group, a naphthyl group, a benzyl group, a furyl group or a thienyl group and the alcohol is represented by the formula:

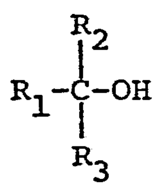

wherein $R_1$, $R_2$ and $R_3$ are each as defined above.

3. A process as claimed in Claim 1, wherein the Grignard reagent is represented by the formula:

$R_1$-MgX

wherein $R_1$ is an arylmethyl group or an alkenyl or alkynyl group having an unsaturated bond at the β-position and X is a halogen atom and the Grignard reaction product is represented by the formula:

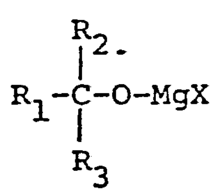

wherein $R_2$ is a hydrogen atom, an alkyl group, an alkenyl group, a phenyl group, a naphthyl group, a benzyl group, a furyl group or a thienyl group, $R_3$ is an alkyl group, an alkenyl group, a phenyl group, a naphtyl group, a benzyl group, a furyl group or a thienyl group and $R_1$ and X are each as defined above.

4. A process as claimed in any one of the preceding claims wherein the inert solvent is a cyclic or non-cyclic ether, optionally together with at least one other solvent inert to the Grignard reaction.

5. A process as claimed in any one of the preceding claims wherein a promoter for the Grignard reaction is additionally supplied to the first reaction zone.

6. A process as claimed in any one of the preceding claims wherein the amount of the organic halide to be supplied to the first reaction zone is from 1 to 20% by weight of the intended amount.

7. A process as claimed in Claim 6 wherein the amount of the organic halide to be supplied to the first reaction zone is from 2 to 10% by weight of the intended amount.

8. A process as claimed in any one of the preceding claims, wherein the first reaction zone comprises a reactor and the second reaction zone comprises at least one reactor.

9. A process as claimed in any one of the preceding claims wherein the first reaction zone and the second reaction zone are respectively maintained at a temperature of from -20°C to the boiling point of the inert solvent.

10. A process as claimed in Claim 9 wherein the first reaction zone and the second reaction zone are, respectively, kept at temperatures in the range of from 0 to 50°C.

11. A process as claimed in any one of the preceding claims wherein the residence times of the reaction mixture in the first reaction zone and in the second reaction zone are, respectively, from 0.2 to 4 hours and from 0.5 to 8 hours.

12. A process as claimed in Claim 11 wherein the residence times of the reaction mixture in the first reaction zone and in the second reaction zone are, respectively, from 0.5 to 2 hours and from 1 to 5 hours.

0073569

European Patent Office

# EUROPEAN SEARCH REPORT

Application number

EP 82 30 3944.1

| DOCUMENTS CONSIDERED TO BE RELEVANT | | | CLASSIFICATION OF THE APPLICATION (Int. Cl. 3) |
|---|---|---|---|
| Category | Citation of document with indication, where appropriate, of relevant passages | Relevant to claim | |
| A | CH - A - 354 066 (METAL & THERMIT)<br>* claim; examples *<br>-- | 1-4 | C 07 C 29/40<br>C 07 C 33/28<br>C 07 C 33/24<br>C 07 C 35/17<br>C 07 D 307/44<br>C 07 D 333/16<br>C 07 B 17/00 |
| A | GB - A - 1 336 140 (NATIONAL RESEARCH DEVELOPMENT)<br>* page 1, lines 8 to 33; page 2, lines 1 to 7 *<br>---- | 1 | |
| | | | TECHNICAL FIELDS SEARCHED (Int.Cl. 3) |
| | | | C 07 B 17/00<br>C 07 C 29/40<br>C 07 C 33/24<br>C 07 C 33/28<br>C 07 C 35/17<br>C 07 D 307/44<br>C 07 D 333/16 |
| | | | CATEGORY OF CITED DOCUMENTS |
| | | | X: particularly relevant if taken alone<br>Y: particularly relevant if combined with another document of the same category<br>A: technological background<br>O: non-written disclosure<br>P: intermediate document<br>T: theory or principle underlying the invention<br>E: earlier patent document, but published on, or after the filing date<br>D: document cited in the application<br>L: document cited for other reasons |
| X | The present search report has been drawn up for all claims | | &: member of the same patent family, corresponding document |

| Place of search | Date of completion of the search | Examiner |
|---|---|---|
| Berlin | 01-11-1982 | KNAACK |

EPO Form 1503.1 06.78